(19) Europäisches Patentamt
European Patent Office
Office européen des brevets

(11) **EP 3 501 439 A1**

(12) **EUROPEAN PATENT APPLICATION**

(43) Date of publication:
**26.06.2019 Bulletin 2019/26**

(21) Application number: **17209947.5**

(22) Date of filing: **22.12.2017**

(51) Int Cl.:
*A61B 18/08* (2006.01)  *A61N 5/06* (2006.01)
*A45D 44/00* (2006.01)  *A61N 1/32* (2006.01)
*A61F 7/00* (2006.01)  *A61B 17/00* (2006.01)
*A61B 18/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Koninklijke Philips N.V.
5656 AE Eindhoven (NL)**

(72) Inventors:
• JURNA, Martin
 **5656 AE Eindhoven (NL)**
• BEIJENS, Linda Goverdina Maria
 **5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **DEVICE AND SYSTEM FOR PERSONALIZED SKIN TREATMENT FOR HOME USE**

(57)     The present invention relates to a mask-shaped skin treatment device providing personalized heat-based skin treatment for home use and a corresponding system. The device comprises one or more temperature sensors configured to measure the skin temperature at one or more skin portions of a user, and one or more heating elements configured to cause local heating of one or more skin portions of the user, wherein the one or more heating elements are configured to cause heat selectively based on the temperature measured by the one or more temperature sensors and wherein the total area of simultaneously activated heating elements ranges from 1 to 5 cm$^2$.

FIG.3

EP 3 501 439 A1

**Description**

FIELD OF THE INVENTION

The present invention relates to a mask-shaped skin treatment device providing personalized heat-based skin treatment for home use. The present invention further relates to a system incorporating the aforementioned device.

BACKGROUND OF THE INVENTION

Skin tightening or firming is a popular cosmetic procedure because it addresses the visible signs of skin aging, including wrinkles and sagging skin. In general, a form of energy (e.g. radio frequency (RF), contact heating, optical energy) is applied to the skin and converted to thermal energy within the skin to raise locally the temperature of the skin. The main target is the dermis and depending on the level of increase in temperature, thermal effects can vary from fibroblast activation, increased collagen production to denaturation and contraction of collagen.

As a result of a mild thermal impact to the dermis, fibroblast stimulation occurs. Fibroblasts are types of cells which synthesize extracellular matrix and collagen as well as the structural framework (stroma) for animal and in particular human tissues. Besides, fibroblasts play a critical role in wound healing. The main function of fibroblasts is to maintain the structural integrity of connective tissues by continuously secreting precursors of the extracellular matrix. Fibroblast stimulation at 40 to 45°C results in collagen remodeling. At higher thermal impact (50 to 90°C) immediate collagen contraction occurs in the dermis followed by collagen remodeling through a controlled wound healing response over time with associated neocollagenesis.

Several skin treatment devices based on especially RF-heating are presently available. According to clinical studies such professional and home-use skin treatment devices show high efficacy in skin tightening and firming. However, in the home-use situation it is difficult for the user to obtain similar results as shown by strictly guided/instructed claim validation tests.

In fact, numerous skin rejuvenation masks using light emitting diodes (LEDs), copper oxide, plant collagen, steam or vibration are commercially available with costs ranging between 30$ till 2500$. Most of these masks provide full facial treatment such that treatment times are usually quite long.

Other home-use devices are handheld devices whose application is cumbersome and which heavily relay on the user's execution of treatment and hence the treatment outcome.

SUMMARY OF THE INVENTION

It is an object of the present invention to provide a skin treatment device that provides easy use, optimal comfort and a treatment time that is as short as possible without having compromises on treatment efficacy.

In a first aspect of the present invention a mask-shaped skin treatment device is presented, the device comprising one or more temperature sensors configured to measure the skin temperature at one or more skin portions of a user, and one or more heating elements configured to cause local heating of one or more skin portions of the user,
wherein the one or more heating elements are configured to cause heat selectively based on the temperature measured by the one or more temperature sensors and wherein the total area of simultaneously activatable heating elements ranges from 1 to 5 cm$^2$.

In a further aspect of the present invention a skin treatment system is presented, the system comprising
a mask-shaped skin treatment device, and
a control unit configured to control the one or more heating elements according to a predefined treatment program and/or according to user instructions.

Preferred embodiments of the invention are defined in the dependent claims. It shall be understood that the claimed system has similar and/or identical preferred embodiments as the claimed device, in particular as defined in the dependent claims and as disclosed herein.

The present invention is based on the idea to provide a skin treatment device that provides optimal comfort, high efficacy and a short treatment time.

The thermal dosage required for mild heating is a combination of time and temperature. There are numerous research papers available showing positive stimulation at temperatures between approximately 40 to 45°C and times from seconds, minutes and even up to hours. The general trend is that having a higher temperature requires a shorter exposure time, and having a lower temperature requires a longer exposure time. The right combination provides the right thermal dosage that allows the fibroblasts within the dermis to be stimulated for collagen remodeling.

Next to the thermal dosage time $t_{therap}$, a particular time is required to reach a specific therapeutic temperature. This time to therapeutic temperature, $t_{rise}$, is a combination between the temperature required for an effective treatment and the heating profile towards this therapeutic selected temperature.

Both the time to therapeutic temperature $t_{rise}$ and the time at therapeutic temperature $t_{therap}$ make up the total treatment time. In general, the higher the therapeutic temperature, the shorter may be the treatment time for an effective treatment. However, a higher therapeutic temperature also implies a longer heating up time. Apart from that, positive treatment effects are only achieved in a range from approximately 40 to 50°C degrees skin temperature. Last but not least, the perception of temperature and hence the experience of comfort is different for every user.

Since the heating elements of the present invention are controlled selectively according to skin temperature the mask-shaped skin treatment device of the present invention offers a way to achieve an overall treatment time, i.e. time to therapeutic temperature plus time at therapeutic temperature, which is as short as possible with respect to a positive treatment effect. In other words, the heat induced in the skin of the user may be adapted to achieve the desired results in a fast and effective manner. Moreover, the heating elements of the proposed skin treatment device are configured to be operated selectively on condition that the total area of simultaneously activated heating elements does not exceed 5 cm². As experiments have shown, this ensures a most comfortable treatment with regard to treatment efficacy and overall treatment duration. Hence, if comfort allows, two or more heating elements may cause heat in the user's skin in parallel as long as the area they cover is not bigger than 5 cm².

Although the heating elements causing heat simultaneously may be located next to each other, they may also be distributed randomly in the device. Hence, skin parts located away from each other may be heated at the same time.

Furthermore, the ratio between heating elements and temperature sensors may vary depending on the skin area to be treated. For example, device parts used to treat skin around eyes may exhibit less heating elements than temperature sensors. Hence, skin temperature in such body regions may be controlled in a closely meshed way to protect the skin. Furthermore, the distance between heating elements and temperature sensors may be low in such device areas to ensure that the temperature measured is the maximum temperature caused by the heating elements. On the other hand, device parts used for less sensitive skin may exhibit more heating elements than temperature sensors.

In an embodiment of the skin treatment device, the one or more heating elements are grouped together as one or more clusters of heating elements, wherein the one or more clusters of heating elements are configured to be activated and/or deactivated sequentially.

In fact, a cluster is a group of simultaneously activatable heating elements. Simultaneously activatable (or activated) heating elements are assigned to a single cluster and may not belong to different clusters. In other words, only heating elements assigned to the same cluster may be activated at the same time such that the maximum size of a cluster corresponds to the total area of simultaneously activated heating elements. If the mask-shaped skin treatment device comprises one hundred heating elements, for example, said heating elements may be grouped into twenty clusters of heating elements, wherein each cluster comprises five heating elements. Since only heating elements corresponding to the same cluster may be activated at the same time, the maximum number of simultaneously activatable/activated heating elements is five. Still, heating elements assigned to different clusters may be activated in a time-shifted manner, in particular sequentially. Apart from that, heating elements of a cluster are not necessarily located adjacent to each other, but may be distributed in any other manner as well.

In another embodiment of the skin treatment device, the one or more heating elements of the one or more clusters are configured to be activated and/or deactivated sequentially, pairwise or according to a predefined pattern. Thus, heating elements within a cluster may be activated at the same time but may also be deactivated. In particular, depending on the skin area to be treated the device may offer one or more customized treatment programs. If the mask-shaped device covers a user's face completely, for example, heating elements on the left hand side may be operated in parallel with mirror-inverted heating elements on the right hand side, which allows a uniform improvement of the user's skin condition. Likewise, if the user has inflamed pimples, for example, the corresponding areas may be excluded from treatment using a predefined program for deactivation.

In another embodiment, the one or more heating elements of the mask-shaped skin treatment device are configured to maintain the temperature they cause if the temperature measured by the one or more temperature sensors corresponds to an adjustable comfort temperature and/or to lower the temperature they cause or to be deactivated if the temperature measured by the one or more temperature sensors exceeds a predefined overheating temperature.

On average, heating the skin to a temperature of about 46°C, for example, leads to skin burn after approximately 45 minutes. In fact, negative effects on the skin may occur at even lower temperature depending on the duration of heat exposure. Apart from possible negative impacts on the skin, user comfort must not be disregarded either. Therefore, the mask-shaped skin treatment device also offers the possibility that a predefined, adjustable comfort temperature may not be exceeded during treatment.

Advantageously, the one or more heating elements cause heat according to a predefined heating profile and/or the total size of simultaneously activatable heating elements depends on an adjustable comfort temperature.

Given a size of 4 cm² of simultaneously activated heating elements, experiments have shown that approximately 75 % of people prefer a fast heating-up profile, whereas 25% of people prefer a longer time for heating-up. According to further experiments using the same heating element size the average comfortable end temperature is 42.3°C with a standard deviation of 0.8°C. It is assumed that a similar spread is relevant for other heating applicator sizes. Preferably, the heating profile of mask-shaped skin firming device complies with said experimental results to ensure user comfort. In particular, the heating profile is represented by an exponential decay of increasing form. More specifically, the temperature profile of the one or more heating elements is given by

$$T(t) = C_1 * \left(1 - e^{-t/\tau}\right) + T_S$$

wherein $C_1$ is the difference between end temperature, i.e. the therapeutic temperature $T_{therap}$ caused in the skin and start temperature $T_S$ of the skin, i.e. $C_1 = T_{therap} - T_S$, $t$ is the heating time and $\tau$ is a time parameter to be determined by experiment. In fact, the parameter $\tau$ decides whether the heating-up phase is long or not, i.e. whether there is a short or fast heating-up phase. The usual offset temperature where the above mentioned heating profile becomes relevant is the skin temperature ranging from 30 to 37°C. Once the skin has been heated up there is an additional time required to stay at the therapeutic temperature, which preferably is the maximum comfortable temperature $T_{max}$ for obtaining the right therapeutic dosage required for an effective treatment. In general, the higher the therapeutic temperature the less time must be spent at said temperature to achieve a desired skin rejuvenation effect.

From experimental results showing that the maximum comfortable temperature $T_{max}$ is related to the total size of simultaneously activated heating elements $A_s$, there has been derived the following formula

$$T_{max} = 0.0194 * A_s^2 - 0.6639 * A_s + 44.644.$$

In the corresponding experiments the skin temperature at start $T_S$ has been 32°C and the mask area has been 10 cm$^2$. Once the temperature $T_{max}$ according to said formula is reached the skin treatment device may maintain the temperature $T_{max}$ caused in the skin.

The time at therapeutic temperature $t_{therap}$ preferably is

$$t_{therap} = e^{\left(\frac{43.061 - T}{0.484}\right)},$$

wherein T is the temperature induced in the user's skin by simultaneously activated heating elements.

In another embodiment, the total area of simultaneously activatable heating elements ranges from 1.5 to 3.5 cm$^2$ in particular from 2.0 to 3.0 cm$^2$. Following the experiments made and the formulas given above the optimum size of skin covered by operating heating elements ranges between 1.5 to 3.5 cm$^2$ given a start temperature of 32°C and an overall mask size, i.e. size covered by heating elements, of 10 cm$^2$. Still, the total area of simultaneously activated heating elements may change with respect to other constraints.

In a further embodiment of the mask-shaped skin treatment device, the one or more heating elements are any one of contact heating elements, absorption-based heating elements and electrically-based heating elements and/or the one or more heating elements (2) are configured to cause heat (201) in a temperature range from 30 to 50°C.

Contact heating elements are Peltier-based heating elements, for example. Under absorption-based heating elements lasers or light emitting diodes (LEDs) are understood. Further suited for skin treatment are Intense Pulsed Light (IPL) systems. Electrically-based heating elements may be radio frequency elements, for example. Unlike lasers, the RF technology produces electric current which generates heat through resistance in the dermis and as deep as subcutaneous fat.

In yet another embodiment, the device is configured to cover the face of the user completely or in parts and/or to cover other body parts and/or to be adapted to a body part contour of the user.

Usually, different users have different problem zones. If a user suffers from periorbital wrinkles, for example, there is no need that the device covers his/her face completely. In such a case it suffices when the device is configured to only cover the eye area. Although of all skin areas facial skin is the most exposed to environmental influences and hence usually requires the most care, there is a trend to treat other skin areas as well. In fact, consumers increasingly focus on treating the skin of neck and hands as well. Hence, the mask-shaped skin treatment device may also be configured to treat said areas.

Apart from that, the mask-shaped skin treatment device may be adapted to a body part contour of the user. For instance, the mask can be configured to be adapted to the shape of the face. The mask may either be provided in a pre-formed manner or formable such that the user may adapt the device form to desired body parts. An adapted mask shape ensures that the distance between heating elements and skin is the approximately the same for all heating elements. In turn, this allows for a uniform treatment result. In particular, the device may be made from flexible material to adapt to the user's skin like a second skin.

In still a further embodiment, the one or more temperature sensors are integrated in the one or more heating elements and/or placed outside the one or more heating elements. Temperature sensors placed directly within the heating elements are highly sensitive to temperature changes induced by the respective elements. Hence, heating elements with integrated temperature sensors may be controlled in a very precise and effective manner. Therefore, these heating elements are preferably used in such areas of the mask which are to be applied to sensitive skin areas. If not integrated in the heating elements the temperature sensors are preferably placed in the vicinity of these elements, in particular adjacent or directly adjacent to said elements. Apart from the location of heating elements the number of temperature sensors should range between one temperature sensor in a cluster and the number of heating elements the mask comprises.

In another embodiment, the one or more temperature sensors are any one of thermistors, thermocouples, resistance temperature detectors, infrared sensors and semiconductor sensors. Above all, thermistors are highly sensitive sensors, low in cost and small in size such that they may fit into the smallest spaces, in particular inside the heating elements. Temperature sensors in the widest sense may further comprise impedance or conductance detectors measuring impedance or conductance, respectively. Although neither impedance nor conductance are directly related to temperature, impedance and conductance allow conclusions about skin temperature. In fact, radio frequency heating elements, for example, are based on impedance heating. Irrespective of the type of temperature sensor used, temperature may either be measured continuously or with interruptions of a predefined length.

In an advantageous embodiment, the mask-shaped skin treatment device further comprises one or more light elements configured to stimulate photobiomodulation of the skin.

In this embodiment, the skin treatment results are further improved. The one or more light elements used may be anyone of lasers, light emitting diodes (LEDs), and broadband light in the visible and near infrared spectrum. The light sources are placed near to or in contact with the skin, allowing the light energy, i.e. photons, to penetrate skin tissue. Consequently, chromophores located in cells of the skin are interacted with resulting in photophysical and photochemical changes that lead to alterations at the molecular, cellular and tissue levels of the body. In particular, wound healing and regeneration of diseased and damaged tissue is accelerated, inflammations are reduced and acute and chronic pain is reduced. Apart from that light may generally enhance performance in normal tissues and accelerate cell cycles resulting in rejuvenation of the skin.

However, photobiomodulation typically requires a long treatment time. In fact, when low cost optical LEDs are used for skin treatment it is assumed that the time required for the right optical dosage is longer than the time required for the thermal based dosage. To provide the right optical dose in this scenario the LEDs are on during the full treatment comprising sequentially activated heating elements, for example. At the end of the treatment both the heating dosage and optical dosage are reached.

Preferably, the mask-shaped skin treatment device further comprises one or more fixation elements configured to fix the device on a body part of the user. On the one hand fixation of the device allows for controlled skin treatment because relocations of the device on the skin are prevented. On the other hand there is no need for the user to hold the device. Thus, the user could do a different activity during treatment (e.g. watching TV, cooking or other house related activities). Accordingly, the suggested device offers skin firming and rejuvenation in an effective and comfortable manner. Fixation elements may be any one of straps, elastic bands, temples, adhesive and other appropriate means.

In another embodiment the mask-shaped skin treatment device further comprises a control unit configured to control the one or more heating elements according to a predefined treatment program and/or according to user instructions, and/or a user interface configured to obtain user instructions from the user and/or to convey information about heating element temperature and/or treatment program, in particular treatment duration.

The predefined treatment program may either be provided by the mask-shaped skin treatment device itself or by the user via the user interface. For example, if the device is configured to treat periorbital wrinkles only, the heating elements may be controlled according to a pre-stored program in every treatment session. If, however, the device is configured to be used for the whole face and to treat dullness, sagging and laxity in addition to wrinkles the user must have the opportunity to choose between different programs or to compose a treatment program of his own. In fact, a treatment program may offer the opportunity to select heating elements and/or clusters to be activated and/or deactivated. This ensures more precise treatment results. Regarding facial skin, for example, areas around eyes, forehead and mouth are usually more affected by skin aging effects and hence require more care than other face areas such as cheeks. Accordingly, skin areas in need of more care may be treated more than areas requiring less care. Hence, the device disclosed also allows for target-oriented treatment.

Accordingly, the user interface may be used by the user to transmit the desired treatment program. The user interface may be a touchscreen, for example, where the user can enter his/her desired maximum comfort temperature, start temperature, program duration etc. The user may also influence the heating profile of the heating elements by choosing a desired speed of heating up. In other words, the user may indicate a preferred $\tau$-value. The user interface may also be a display informing the user about the current treatment program or showing the heating profile. Likewise, the user may be informed about treatment beginning and ending via an acoustic signal.

In an embodiment of the skin treatment system, the system further comprises a user interface configured to obtain user instructions from the user and/or to convey information about any one of heating element temperature and/or treatment program, in particular treatment duration. The user interface may be a smartphone, for example, wherein the heating element temperature and treatment duration are displayed on the smartphone's display.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of a first embodiment of a mask-shaped skin treatment device according to the present invention,

Fig. 2 shows a schematic diagram of a second embodiment of a mask-shaped skin treatment device according to the present invention,

Fig. 3 shows a side view of an exemplary implementation of a first embodiment of a mask-shaped skin treatment system according to the present invention,

Fig. 4 shows a top view of an exemplary implementation of a second embodiment of a mask-shaped skin treatment system according to the present invention,

Figs. 5A, 5B and 5C show each a front view of an exemplary implementation of an embodiment of a mask-shaped skin treatment device according to the present invention,

Figs. 6A, 6B and 6C show different embodiments of heating elements according to the present invention,

Fig. 7 shows a heating profile of a heating element according to the present invention,

Fig. 8 shows the effective stimulation correlation between temperature and treatment duration,

Fig. 9 show the relation between the maximum comfortable temperature and the size of simultaneously activated heating elements as measured with a mask 1 comprising Peltier heating elements,

Figs. 10A to 10E each show a heating profile for a heating element as desired by a volunteer,

Fig. 11 shows the distribution of the parameter $\tau$ in units of seconds as derived from several experiments,

Fig. 12 shows the solution space for comfortable effective treatment with respect to temperature of the heating elements, treatment time and maximum comfortable temperature,

Fig. 13 shows the solution space for comfortable effective treatment with respect to temperature of the heating elements, treatment time and parameter $\tau$,

Fig. 14 shows the solution space for comfortable effective treatment with respect to temperature of the heating elements, treatment time and efficacy level,

Fig. 15 shows the relation between the temperature caused by the heating elements and the total area of simultaneously activated heating elements. Fig. 16 shows the distribution of temperature T

Fig. 17 shows the distribution of the parameter $\tau$,

Fig. 18 shows the relation between the total treatment time $t_{total}$ and the total size of simultaneously activated heating elements $A_s$ needed to acquire an effective treatment.

Fig. 19 shows a histogram of the optimal total area of heating elements causing heat simultaneously,

Fig. 20 shows a histogram of the optimum treatment time, and

Fig. 21 shows the correlation between total treatment time and total area of heating elements causing heat simultaneously which is required for an effective treatment..

DETAILED DESCRIPTION OF THE INVENTION

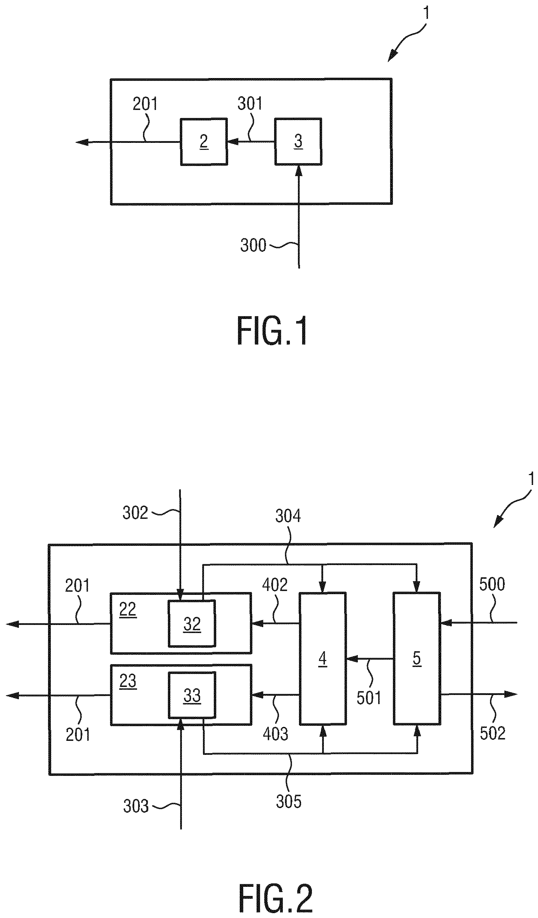

Fig. 1 shows a schematic diagram of an embodiment of a mask-shaped skin treatment device 1 according to the present invention. The device 1 comprises a heating element 2 and a temperature sensor 3.

The heating element 2 of this embodiment is a Peltier elements having a size of 3 cm$^2$. After measuring a user's skin temperature, i.e. detecting his/her skin temperature signal 300, the temperature sensor 3 transmits the corresponding skin temperature data 301 to the heating element 2. Based upon the data 301 obtained the heating element 2 causes heat 201 in the skin of the user by contact heating. If the skin temperature data 301 indicate that a predefined threshold temperature of 50°C is exceeded, the heating element stops heating. If the temperature measured is within the range of 45 to 50°C the heating element reduces its heat and if the temperature measured is below 40°C the heating element is heated up further. Only if the temperature indicated by the temperature sensor 3 is within 40 to 45°C the heat produced by the Peltier element 2 remains constant. In other words, there exists a control loop or feedback loop between the heating element 2 and the temperature sensor 3. Based on the temperature sensor reading the heating element 2 is actively controlled such that the heating element 2 has the right set temperature to achieve better skin quality by stimulating fibroblasts. Hence, based on the temperature measured by the temperature sensor the heating element either heats up the user's skin further, maintains the heat it causes in the skin or stops heating.

Fig. 2 shows a schematic diagram of a second embodiment of a mask-shaped skin treatment device 1 according to the present invention. Apart from two heating elements 2 and two temperature sensors 3 each integrated in the heating elements 2, this embodiment comprises a control unit 4 configured to control the two heating elements and a user interface 5. Using the user interface 5 the user may choose between several treatment programs. In particular, the user may select particular heating elements to become part of a cluster and/or select clusters of heating elements to be used for treatment. Having received the corresponding user instructions 500, the user interface 5 transmits the corresponding instruction data 501 to the control unit 4. Subsequently, the control unit 4 transmits control signals 400 to control the heating elements 2 according to said instructions data 501. Accordingly, the heat 201 of the heating elements 2 induced in the skin of the user conforms with the user instructions 500. To control that the heating elements 2 work as desired the skin temperature is measured by the temperature sensors 3 integrated within the heating elements 2. The measurement results 301 are then transmitted to the control unit 4, which is configured to control the heating elements 2 in accordance. The measurement results 301 of each sensor 3 are furthermore transmitted to the user interface which presents the data 301 as user information 501 to the user.

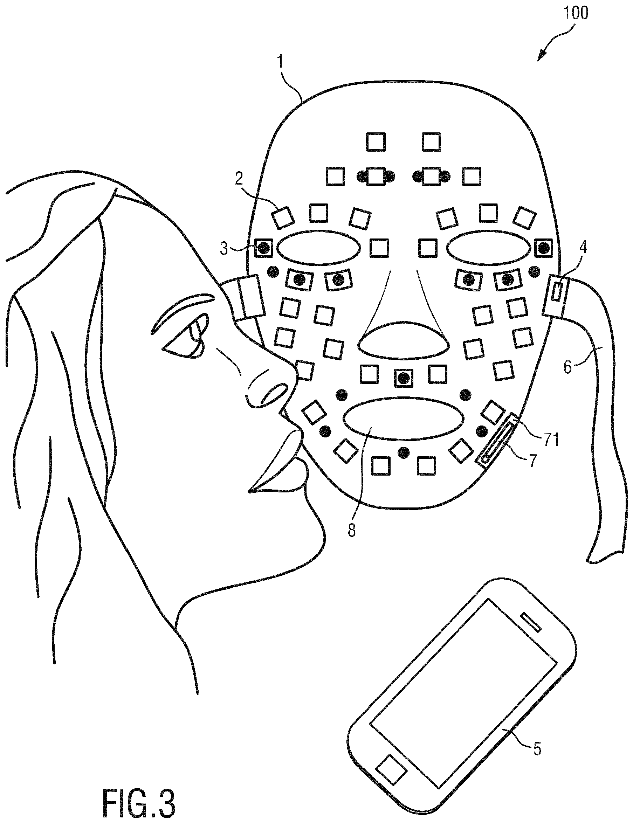

Fig. 3 shows a side view of an exemplary implementation of a first embodiment of a mask-shaped skin treatment system 100 according to the present invention. The system 100 comprises an embodiment of a mask-shaped skin treatment device 1 configured as full-face mask and a user interface 5 in the form of a smartphone. The mask 1 comprises multiple heating elements 2 and temperature sensors 3. In addition, the mask comprises a control unit 4 and a lashing strap as fixation element 6. The heating elements 2, temperature sensors 3 and the control unit 4 are operated by a battery 7 comprised within a battery compartment 71. To enhance user comfort the mask 1 further comprises holes 8 for eyes, nose and mouth.

The heating elements 2 in this embodiment are rectangular Peltier elements providing contact heat to the skin. The number of temperature sensors 3 varies among different mask areas. Mask areas to be applied to more sensitive skin areas are covered by more temperature sensors 3 than areas corresponding to less sensitive skin areas. Hence, the area applied to the forehead and around the eyes and mouth is covered by more temperature sensors 3 than the mask area used for the cheeks, for example. For more precise measurements, temperature sensors 3 used to measure the skin temperature in more sensitive skin areas are integrated in the heating elements 2 while other temperature sensors 3 are placed outside the heating elements 2. The control unit 4 controlling the heating elements 2 is configured to receive user instructions via the user interface 5. In this embodiment, the control unit 4 may receive said information wirelessly.

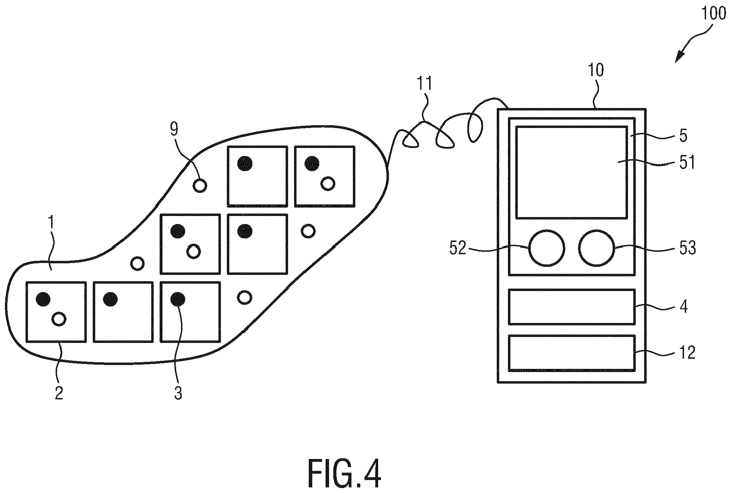

Fig. 4 shows a top view of an exemplary implementation of a second embodiment of a mask-shaped skin treatment system 100 according to the present invention. This embodiment of a mask-shaped treatment system 100 comprises a mask-shaped skin treatment device 1 in the form of a comprising several heating elements 2, temperature sensors 3 and light elements 9 which is connected via a cable 11 to a base station 10 comprising a user interface 5, a control unit 4 and a storage unit 12. The user interface 5 comprises a display 51 and two selection buttons 52 and 53.

The device 1 may work in two modes: an initialization mode and an operating mode. In the initialization mode the user may determine his/her personal maximum comfortable end temperature $T_{max}$ and the speed or time in which said temperature shall be reached. In order to determine said quantities a cluster of heating elements 2 causes increasing heat 201 in the skin. However, the user may correct the pre-set temperature of said clusters himself/herself for a predefined time using the user interface 5. For example, he/she may control the setting using the selection buttons 52 and 53. The only constraint the user has to comply with is that his/her perception of a respective temperature is comfortable. In fact, $T_{max}$ may be skin area dependent such that the user may determine skin area or body portion specific values for $T_{max}$. The data corresponding to the temperature profile(s) of the user are stored in the storage unit 12. After expiration of the predefined time the control unit 4 computes the maximum comfortable temperature(s) and preferred rise time(s) or speed(s) from the temperature profile(s) obtained and the storage unit 12 stores the corresponding data as well. Once one or more personalized $T_{max}$ and parameter $\tau$ values are determined, the device may switch to the operating mode and take into account the corresponding data for upcoming treatment sessions. However, the control unit 4 of the device 1 may likewise control the heating elements 2 according to default parameters stored in the storage unit 12.

In the operating mode the embodiment is configured to provide skin treatment using thermal and photobiomodulation effects in the human skin. Light elements 9 and heating elements 2 may be activated simultaneously, sequentially or according to a predefined program. Predefined programs for the individual operation of heating elements 2 and light elements 9 or their collaboration may be stored in the storage unit 12. The programs available may be displayed on the display of the base station 10 and the user may choose a particular program using the selection buttons 52 and 53. Upon selection of a program the control unit 4 may control the temperature caused by the heating elements and the light emitted by the one ore more light elements 9 in accordance with said program. In particular, the control unit 4 controls the intensity of light provided by the light elements. However, the control unit 4 may likewise control the wavelength at which the light elements are operating. Furthermore the control unit 4 may instruct the heating elements cluster wise such that different clusters may cause heat according to different values of $T_{max}$ and $\tau$ as defined by the user.

The mask-shaped skin treatment device 1 is given by a flexible patch comprising adhesive and hence may be attached to any region of the user's body. Furthermore, the base station 10 is configured to be small enough to be stored in a trouser pocket of the user. Hence the user may even do sports during treatment.

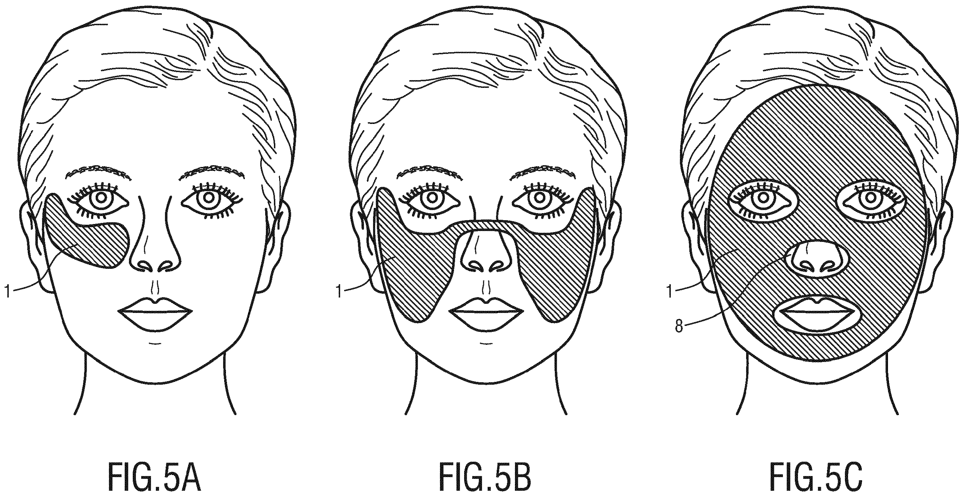

Figs. 5A, 5B and 5C show each a front view of an exemplary implementation of an embodiment of a mask-shaped skin treatment device 1. While Fig. 5A shows an embodiment of a device 1 that solely covers the lower eye region of the user, Fig. 5B shows an embodiment that covers both cheeks and Fig. 5B shows an embodiment which covers the face of the user completely apart from his/her eyes, nose and mouth. The embodiment solely covering the lower eye region is particularly suited for skin rejuvenation below the eyes and to treat eye bags. With the embodiment shown in Fig. 5B wrinkles on cheeks may be treated effectively. The mask 1 as shown in Fig. 5C offers a full-face treatment. Holes 8 inside the mask increase user comfort.

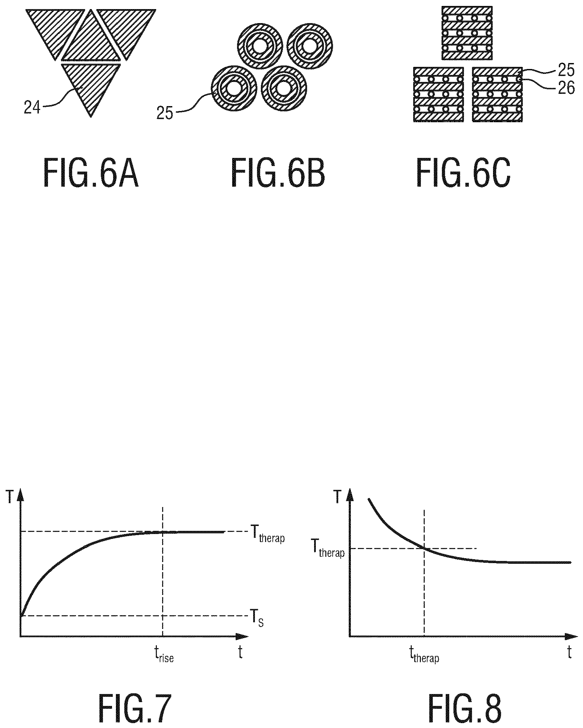

Figs. 6A, 6B and 6C show different embodiments of heating elements 2 according to the present invention. Fig. 6A shows four triangular-shaped Peltier heating elements 21. Fig. 6B shows a radio frequency configuration, in particular four centrosymmetric radio frequency electrodes 22. Fig. 6C shows a squared heating element created from different line shaped radio frequency electrodes 22, where in between the electrodes 22 LEDs 23 are located. The LEDs 23 may either be used for inducing heat or to offer an additional photobiomodulation effect.

Fig. 7 shows a heating profile of a heating element 2 according to the present invention. In particular, Fig. 7 shows the temperature T caused by the heating elements 2 with respect to heating time t. The profile corresponds to exponential decay of increasing form. The total heating time can be divided into two parts: the time to reach therapeutic temperature, i.e. the heating-up time, and the time at therapeutic temperature (thermal dosage time). The time range $t_{rise}$ is the heating-up time, in which the temperature rises up from a value $T_S$ to the therapeutic temperature $T_{therap}$ which preferably is the maximum comfortable temperature $T_{max}$. After $t_{rise}$ the temperature induced by the heating elements 2 remains constant during thermal dosage time $t_{therap}$.

Fig. 8 shows the effective stimulation correlation between temperature and treatment duration. To achieve an effective treatment at least a duration of $t_{therap}$ is required at a temperature that is as high as the maximum comfortable temperature $T_{max}$.

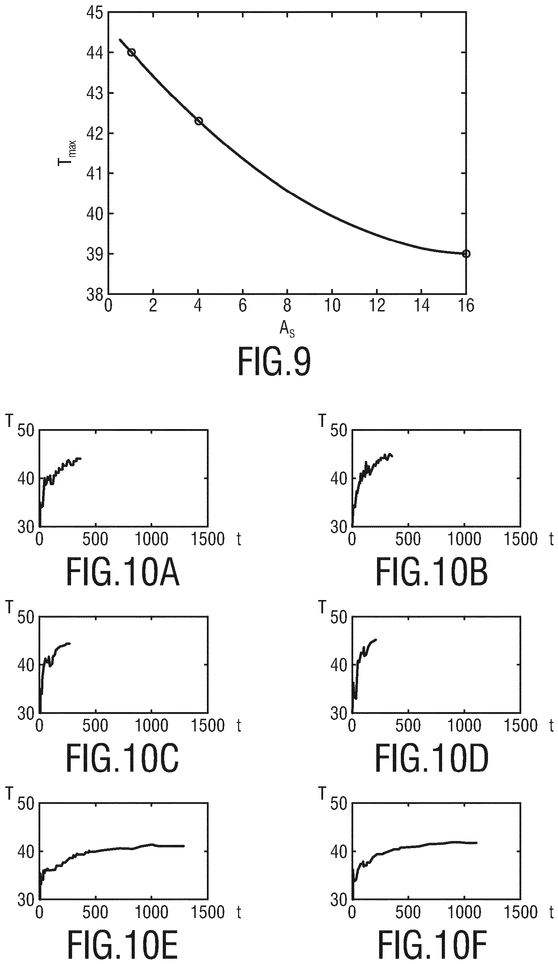

Fig. 9 shows the relation between the maximum comfortable temperature $T_{max}$ and the size $A_s$ of simultaneously activated heating elements as measured with a mask 1 comprising Peltier heating elements. $T_{max}$ is given in units of °C and size $A_s$ is given in units of cm². To achieve the same treatment effect, a larger size of simultaneously heated skin allows for lower heating temperature, whereas a smaller cluster size allows for higher temperatures. For example, Peltier elements covering 1 cm² of the user's skin may heat up to 44°C. Peltier elements, on the other hand, covering 16 cm² may only be heated up to 39°C. Otherwise, the user would feel uncomfortable. In fact, to achieve reasonable treatment results without compromising user's comfort the total area of simultaneously activated heating elements should range from 1 to 5 cm².

Figs. 10A to 10F each show a heating profile for a heating element 2 as desired by a volunteer. Each of the figures show the temperature T of the heating elements with respect to heating time t. While T is given in units of °C, the time t is given in units of seconds. It can be seen that the volunteers have to change the temperature setting of the heating elements regularly to constantly feel comfortable. The heating profile desired by all volunteers can roughly be fitted by an exponential decay function.

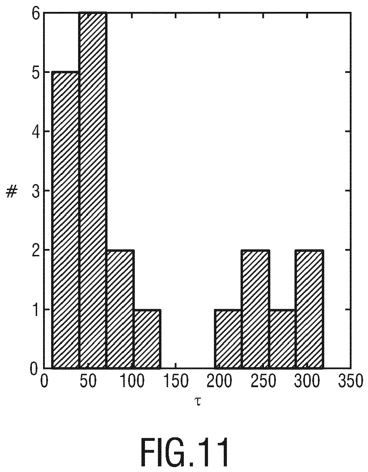

Fig. 11 shows the distribution of the parameter $\tau$ in units of seconds as derived from several experiments and using the formulas above.

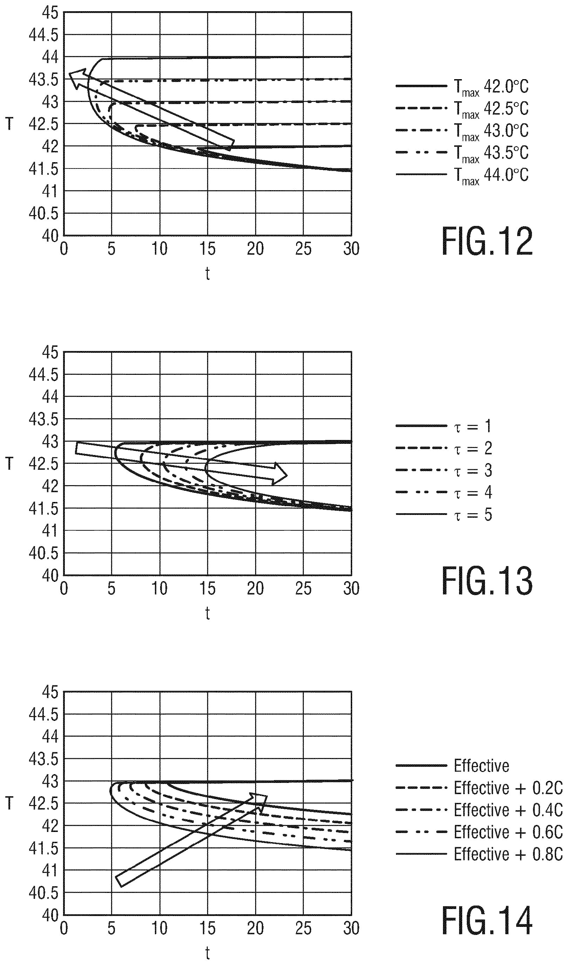

Fig. 12 shows the solution space for comfortable effective treatment with respect to the temperature T caused by the heating elements 2, treatment time t (i.e heating-up time and time at maximum comfortable temperature) and the maximum comfortable temperature $T_{max}$ of a user. The relations shown are based on experimental input. In the graph depicted, the units of T and $T_{max}$ are given in °C and the units for treatment time are given in minutes. Generally, the higher the maximum comfortable temperature $T_{max}$ of a user, the shorter may be the time to achieve an effective treatment. For example, if the user's maximum comfortable temperature is as high as 43°C or even above this value, he/she may already achieve a positive effect of treatment in less than 5 minutes. Furthermore, it can be seen that the higher the maximum comfort temperature of the user, the bigger the temperature range for an effective treatment, i.e. for stimulating fibroblasts. In an embodiment of the mask-shaped skin firming device the control unit 4 may control the heating elements according to this relationship between temperature range applicable, treatment time and maximum comfort temperature. Provided a maximum comfortable temperature associated with the user, the device may select an appropriate treatment time to achieve optimal treatment results. Furthermore, data corresponding to the relationship between the user's maximum comfortable temperature, the temperature caused by the heating elements in the skin and treatment may be stored in a storage unit 12, wherein the control unit 4 has access to said storage unit 12.

Fig. 13 shows the solution space for comfortable effective treatment with respect to temperature T of the heating elements 2, treatment time t and the parameter $\tau$. The relations shown are based on experiments. In the graph depicted, the units for T are given in °C and the units for t and $\tau$ are given in minutes. The smaller the value of $\tau$, i.e. the faster heat is provided, the shorter does an effective and comfortable treatment have to last. For $\tau = 1$, for example, an effective treatment may only last slightly above 5 minutes if the temperature $T$ the heating elements are supposed to reach is chosen to be between 42.5 and 43°C. The data comprised in the given graph may be stored in a storage unit 12 of the device 1 or system 100. Furthermore, the control unit 4 may control the heating elements according to said data.

Fig. 14 shows the solution space for comfortable effective treatment with respect to temperature $T$ of the heating elements 2, treatment time $t$ and efficacy level according to experiment. The units of $T$ are given in °C and the units for t are given in minutes. In general, the higher the temperature $T$ caused by the heating elements and longer the treatment time, the more effective the treatment proves to be. However, above a temperature threshold of approximately 50°C the treatment is no longer effective irrespective of the treatment time $t$. The data comprised in the given graph may be stored in a storage unit 12 of the device 1 or system 100. Furthermore, the control unit 4 may control the heating elements according to said data.

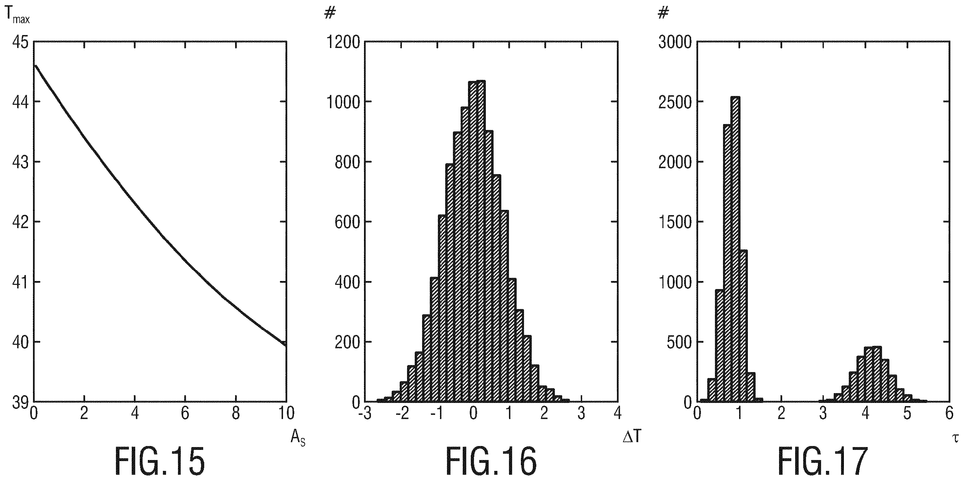

Fig. 15 shows the relation between temperature $T$ caused by the heating elements 2 and the total area $A_s$ of simultaneously activated heating elements, i.e. the maximum cluster size. The histogram shown is based on a Monte Carlo simulation using 10.000 iterations. The simulation is based on knowledge derived from several experiments and corresponding to the above given formulas. In particular, the starting temperature of the skin is assumed to be 32 °C and the mask area, i.e. device area configured to cover the user's skin, is assumed to be 10 cm$^2$. The temperature increase profile corresponding to a temperature increase perceived as comfortable is given by $\tau = 0.75 * (0.84 \pm 0.2) + 0.25 * (4.16 \pm 0.4)$ in units of minutes as derived from Fig. 11. In this profile the standard deviation for $\tau = 0.84$ is 0.2 and for $\tau = 4.16$ is 0.4. As can be seen from Fig. 15 with a total area $A_s$ of simultaneously activated heating elements corresponding to 1 cm$^2$ the therapeutic temperature may be as high as 44 °C roughly. However, assuming that indeed 10 cm$^2$ have to be treated, the overall treatment time is $t_{total} = 10 * (t_{rise} + t_{therap})$. On the other hand, if $A_s$ is 5 cm$^2$, the therapeutic temperature perceived as comfortable may be 41.7 °C such that the total treatment time is $t_{total} = 2 * (t_{rise} + t_{therap})$. Although the time at therapeutic temperature $t_{therap}$ takes longer the lower the temperature is at which a respective treatment is performed, the less time is spent on heating up the device. Fig. 16 shows the distribution of the maximum comfortable temperature $T_{max}$ in °C as derived from a Monte Carlo simulation using 10.000 iterations. The simulation is based on experiments and the formulas given above.

Fig. 17 shows the distribution of the parameter $\tau$ representing the heating-up time of the heating elements based on a Monte Carlo simulation with 10.000 trials, $\tau$ is given in minutes in Fig. 17. Like the simulations depicted in Figs. 15 and 16, the simulation is based on experiments and the formulas given above. In particular, the constraints with respect to mask size, start temperature and standard variations are the same for all simulations performed.

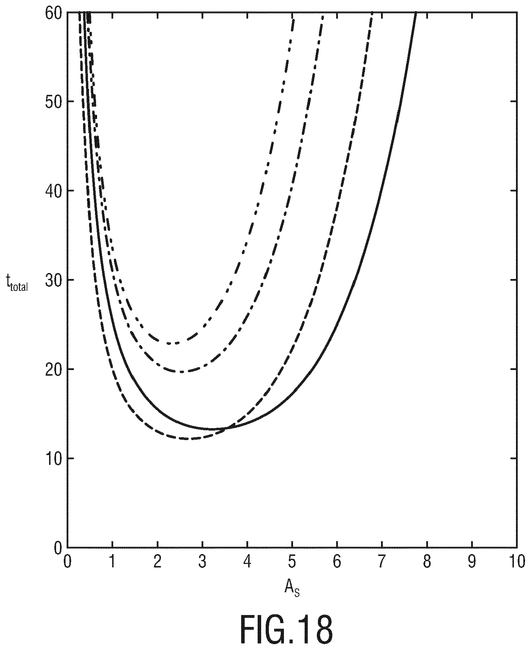

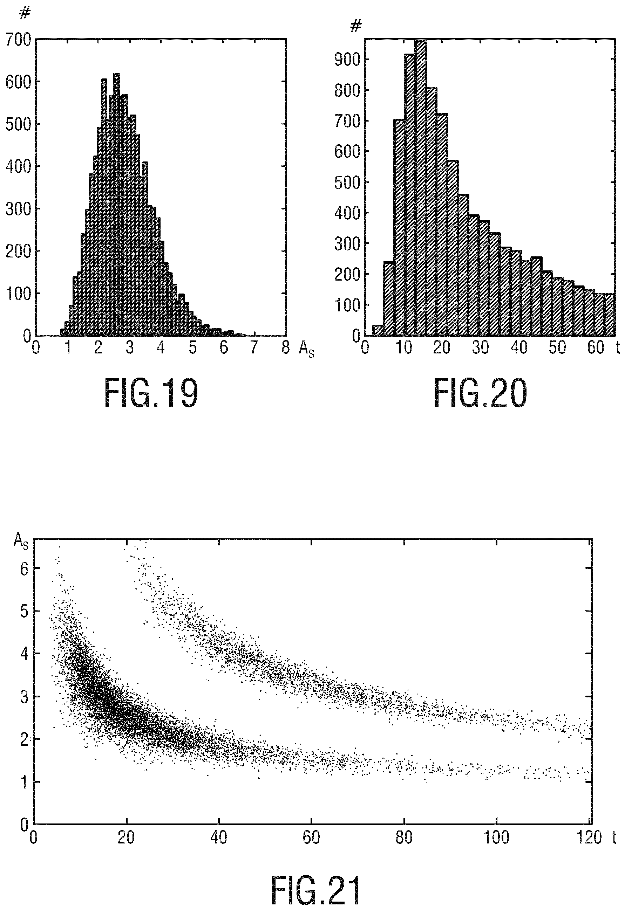

Fig. 18 shows the relation between the total treatment time $t_{total}$ and the total size of simultaneously activated heating elements $A_s$ needed to acquire an effective treatment. In fact, the graph shows four Monte Carlo simulations using four different parameter sets with $T_{max}$ and $\tau$ as parameters. The constraints on start temperature $T_s$ and mask size are given by 32 °C and 10cm$^2$, respectively. In general, the treatment time is a combination of the heating-up time $t_{rise}$ and the time at therapeutic temperature $t_{therap}$. Preferably, the temperature the heating elements cause in the skin during treatment is the maximum comfortable temperature $T_{max}$ of the user. Following Fig. 12, the higher $T_{max}$, the shorter the time at therapeutic temperature. However, the time period for heating up is prolonged the higher $T_{max}$. Furthermore, according to Fig. 15 for example, the $T_{max}$ is dependent on the area of heating elements causing heating at the same time. Accordingly, for an effective treatment at a given maximum comfortable temperature there is exists an area of simultaneously activated heating elements at which the total treatment time is shortest. Regarding Fig. 18 said optimum area $A_s$ ranges between 2 and 4cm$^2$, In fact, for the most parameter sets the optimum size corresponding to simultaneous operation of heating elements is 2 to 3cm$^2$. Fig. 19 shows a histogram of the optimal total area of heating elements causing heat simultaneously. The histogram is based on a Monte Carlo simulation using 10.000 iterations. The optimum total area of heating elements causing heat simultaneously, $A_s$, is given in units of cm$^2$. For a large number of parameters, the optimum size of skin subject to heat by the heating elements turns out to be between 2 and 3cm$^2$.

Fig. 20 shows a histogram of the optimum treatment time (i.e. time to reach the maximum comfortable temperature and the time at the maximum comfortable temperature) for a given size of simultaneously heated skin. The histogram is based on a Monte Carlo simulation using 10.000 iterations. The optimum treatment time is given in minutes and has a peak at approximately 15 minutes.

Fig. 21 shows the correlation between total treatment time $t_{total}$ and total area $A_s$ of heating elements causing heat simultaneously which is required for an effective treatment. The total area $A_s$ of simultaneously activated heating elements is given in cm$^2$ and the total treatment time $t$ is given in minutes. The correlation is based on a Monte Carlo simulation using 10.000 different parameter sets. For a mask with heating elements covering 10cm$^2$ and a start temperature of 32°C, an efficient treatment allowing for a minimal treatment time is achieved for size $A_s$ ranging between 1 and 5cm$^2$, preferably between 1.5 and 3.5cm$^2$, and even more preferably between 2 and 3cm$^2$

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1.   A mask-shaped skin treatment device (1) comprising
one or more temperature sensors (3) configured to measure the skin temperature at one or more skin portions of a user, and
one or more heating elements (2) configured to cause local heating (201) of one or more skin portions of the user,
wherein the one or more heating elements (2) are configured to cause heat selectively based on the temperature measured by the one or more temperature sensors (3) and wherein the total area of simultaneously activatable heating elements (2) ranges from 1 to 5 cm$^2$.

2.   The mask-shaped skin treatment device (1) according claim 1, wherein the one or more heating elements (2) are grouped together as one or more clusters of heating elements, wherein the one or more clusters of heating elements are configured to be activated and/or deactivated sequentially.

3.   The mask-shaped skin treatment device (1) according to claim 2,
wherein the one or more heating elements (2) of the one or more clusters are configured to be activated and/or deactivated sequentially, pairwise or according to a predefined pattern.

4.   The mask-shaped skin treatment device (1) according to any one of the preceding claims, wherein the one or more heating elements (2) are configured to maintain the temperature they cause if the temperature measured by the one or more temperature sensors corresponds to an adjustable comfort temperature and/or to lower the temperature they cause or to be deactivated if the temperature measured by the one or more temperature sensors (3) exceeds a predefined overheating temperature.

5.   The mask-shaped skin treatment device (1) according to any one of the preceding claims, wherein the one or more heating elements (2) cause heat according to a predefined heating profile and/or wherein the total size of simultaneously activatable heating elements (2) depends on an adjustable comfort temperature.

6.   The mask-shaped skin treatment device (1) according to any one of the preceding claims, wherein the total area of simultaneously activatable heating elements (2) ranges from 1.5 to 3.5 cm$^2$, in particular from 2.0 to 3.0 cm$^2$.

7.   The mask-shaped skin treatment device (1) according to any one of the preceding claims, wherein the one or more heating elements (2) are any one of contact heating elements, absorption-based heating elements and electrically-based heating elements and/or wherein the one or more heating elements (2) are configured to cause heat (201) in a temperature range from 30 to 50°C.

8.   The mask-shaped skin treatment device (1) according to any one of the preceding claims, wherein the device (1) is configured to cover the face of the user completely or in parts and/or to cover other body parts and/or wherein the device (1) is configured to be adapted to a body part contour of the user.

9.   The mask-shaped skin treatment device (1) according to any one of the preceding claims, wherein the one or more temperature sensors (3) are integrated in the one or more heating elements (2) and/or placed outside the one or more heating elements (2).

10.  The mask-shaped skin treatment device (1) according to any one of the preceding claims, wherein the one or more temperature sensors (3) are any one of thermistors, thermocouples, resistance temperature detectors, infrared sensors and semiconductor sensors.

11. The mask-shaped skin treatment device (1) according to any one of the preceding claims, further comprising one or more light elements (9) configured to stimulate photobiomodulation of the skin.

12. The mask-shaped skin treatment device (1) according to any one of the preceding claims, further comprising one or more fixation elements (6) configured to fix the device (1) on a body part of the user.

13. The mask-shaped skin treatment device (1) according to any one of the preceding claims, further comprising
a control unit (4) configured to control the one or more heating elements according to a predefined treatment program and/or according to user instructions, and/or
a user interface (5) configured to obtain user instructions from the user and/or to convey user information about heating element temperature and/or treatment program, in particular treatment duration.

14. A skin treatment system (100) comprising
a mask-shaped skin treatment device (1) according to claims 1 to 12, and
a control unit (4) configured to control the one or more heating elements according to a predefined treatment program and/or according to user instructions.

15. The skin treatment system (100) according to claim 14,
further comprising a user interface (5) configured to obtain user instructions from the user and/or to convey information about any one of heating element temperature and/or treatment program, in particular treatment duration.

FIG.1

FIG.2

FIG.3

FIG.4

EP 3 501 439 A1

FIG.5A  FIG.5B  FIG.5C

24

25

25
26

FIG.6A          FIG.6B          FIG.6C

$T_{therap}$

$T_S$

$t_{rise}$          t

FIG.7

$T_{therap}$

$t_{therap}$          t

FIG.8

FIG.9

FIG.10A

FIG.10B

FIG.10C

FIG.10D

FIG.10E

FIG.10F

FIG.11

FIG.12

FIG.13

FIG.14

FIG.15

FIG.16

FIG.17

FIG.18

FIG.19

FIG.20

FIG.21

Europäisches
Patentamt

European
Patent Office

Office européen
des brevets

# EUROPEAN SEARCH REPORT

Application Number

EP 17 20 9947

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/052986 A2 (SYNERON MEDICAL LTD [IL]; ECKHOUSE SHIMON [IL]; FLYASH LION [IL]) 26 April 2012 (2012-04-26)<br>* figures 1,2 *<br>* paragraphs [0008], [0029] - [0034] *<br>----- | 1-15 | INV.<br>A61B18/08<br>A61N5/06<br>A45D44/00<br>A61N1/32<br>A61F7/00 |
| X | EP 2 889 053 A1 (PANASONIC IP MAN CO LTD [JP]) 1 July 2015 (2015-07-01)<br>* paragraphs [0029], [0061] - [0062], [0109] - [0112], [0185], [0133] *<br>* figures 1,2,6-10 *<br>----- | 1-4,6-15 | A61B17/00<br>A61B18/00 |
| A | DE 20 2008 006017 U1 (BRAU INGE [DE]) 10 July 2008 (2008-07-10)<br>* figures 1-2 *<br>----- | 1-15 | |
| A | WO 2012/073232 A1 (SYNERON MEDICAL LTD [IL]; ECKHOUSE SHIMON [IL]; FLYASH LION [IL]) 7 June 2012 (2012-06-07)<br>* figures 4a-c *<br>* paragraph [0032] *<br>----- | 1-15 | |
| A | US 2015/328474 A1 (FLYASH LION [IL] ET AL) 19 November 2015 (2015-11-19)<br>* paragraphs [0056], [0066] *<br>----- | 9 | TECHNICAL FIELDS SEARCHED (IPC)<br>A61B<br>A61N<br>A45D<br>A61F |

The present search report has been drawn up for all claims

| Place of search | Date of completion of the search | Examiner |
|---|---|---|
| The Hague | 24 May 2018 | Husselin, Stephane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 20 9947

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2018

| Patent document cited in search report | | Publication date | Patent family member(s) | | | Publication date |
|---|---|---|---|---|---|---|
| WO 2012052986 | A2 | 26-04-2012 | EP | 2627400 | A2 | 21-08-2013 |
| | | | WO | 2012052986 | A2 | 26-04-2012 |
| EP 2889053 | A1 | 01-07-2015 | CN | 104740760 | A | 01-07-2015 |
| | | | EP | 2889053 | A1 | 01-07-2015 |
| | | | JP | 2015142718 | A | 06-08-2015 |
| | | | US | 2015182737 | A1 | 02-07-2015 |
| DE 202008006017 | U1 | 10-07-2008 | DE | 112008003934 | A5 | 07-04-2011 |
| | | | DE | 202008006017 | U1 | 10-07-2008 |
| | | | WO | 2009132606 | A1 | 05-11-2009 |
| WO 2012073232 | A1 | 07-06-2012 | EP | 2646103 | A1 | 09-10-2013 |
| | | | JP | 6040159 | B2 | 07-12-2016 |
| | | | JP | 2013544175 | A | 12-12-2013 |
| | | | KR | 20130136487 | A | 12-12-2013 |
| | | | US | 2013274841 | A1 | 17-10-2013 |
| | | | WO | 2012073232 | A1 | 07-06-2012 |
| US 2015328474 | A1 | 19-11-2015 | CN | 103945786 | A | 23-07-2014 |
| | | | EP | 2782512 | A1 | 01-10-2014 |
| | | | JP | 6078550 | B2 | 08-02-2017 |
| | | | JP | 2015501695 | A | 19-01-2015 |
| | | | KR | 20140096267 | A | 05-08-2014 |
| | | | US | 2015328474 | A1 | 19-11-2015 |
| | | | WO | 2013076714 | A1 | 30-05-2013 |

EPO FORM P0459

For more details about this annex : see Official Journal of the European Patent Office, No. 12/82